# EUROPÄISCHE PATENTSCHRIFT

(11) **EP 2 934 384 B1**
(45) Veröffentlichungstag und Bekanntmachung des Hinweises auf die Patenterteilung: **04.10.2017**
(21) Anmeldenummer: 13818661.4
(22) Anmeldetag: 16.12.2013
(51) Int. Cl.: A61F 2/20, A61M 16/04

(54) **SPRECHVENTIL**
SPEAKING VALVE
VALVE DE PHONATION

(30) Priorität: 19.12.2012 DE 102012024817
(43) Veröffentlichungstag der Anmeldung: 28.10.2015
(73) Patentinhaber: Andreas Fahl Medizintechnik-Vertrieb GmbH, 51149 Köln (DE)
(72) Erfinder: FAHL, Andreas, 50676 Köln (DE)
(74) Vertreter: Geskes, Christoph
(86) Internationale Anmeldenummer: PCT/EP2013/003790
(87) Internationale Veröffentlichungsnummer: WO 2014/095024

(56) Entgegenhaltungen:
- WO-A1-2010/060983
- DE-U1-202011 003 781
- US-A1- 2003 029 456

## Beschreibung

Die vorliegende Erfindung betrifft ein Sprechventil für Lanryngektomierte oder Tracheotomierte mit einem Gehäuse.

Bei operativen Eingriffen im oberen Atemtrakt kann das Anlegen einer künstlichen Atemöffnung (Tracheostoma) in der Luftröhre notwendig sein, damit unter Umgehung von Mundraum und Kehlkopf Luft direkt in die Lunge eingeamtet werden kann. Bei Laryngektomierten oder Tracheotomierten werden üblicherweise Filtersysteme eingesetzt, welche aus einem Pflaster mit einem eingesetzten Filter, aus einer üblicherweise selbstklebenden Basisplatte oder einer Trachealkanüle bestehen - in aller Regel aus Kunststoff gebildet -, in welche Filter unterschiedlichster Art einsetzbar sind, bestehen. Zu den Filtersystemen, welche für laryngo-tracheale Hilfsmittel wie Trachealkanülen und Pflaster- oder Basisplatte eingesetzt werden, zählen die sogenannten Feuchte- und Wärmeaustauscher, auch künstliche Nasen genannt. Diese dienen dazu, die bei tracheotomierten, aber auch bei laryngektomierten Patienten fehlenden Regulationsmechanismen zur Erwärmung und Befeuchtung der Atemluft nachzubilden und ein Inkontaktbringen der Luftröhre mit trockner, kalter und nichtgefilterter Luft zu vermeiden. Denn durch die hierdurch hervorgerufene Reizung tritt eine erhöhte Schleimproduktion mit der nachfolgenden Gefahr der Verborkung auf. Durch Feuchte- und Wärmeaustauscher wird nun die eingeatmete Luft angefeuchtet, erwärmt und gleichzeitig gefiltert. Hierdurch wird die vorstehend erwähnte Borkenbildung weitgehend vermieden. Dabei hilft das regelmäßige Tragen der künstlichen Nase insbesondere bei starker Sekretabsonderung, da durch das Anfeuchten der Schleimhäute in der Luftröhre die Sekretproduktion vermindert wird. Feuchte- und Wärmeaustauscher können darüber hinaus auch mit einer Sprechfunktion ausgestattet sein, und werden dann, so auch im Sinne der vorliegenden Erfindung, als Sprechventil angesprochen, wobei jedoch andere Bezeichnungen wie Beispiel Stimmventil ebenfalls gängig sind.

Gattungsgemäße Sprechventile sind aus dem Stand der Technik vielseitig bekannt. So offenbart die DE 699 20 440 T2 eine gattungsgemäße, dort als Stimmventil mit Filter bezeichnete Vorrichtung, wobei das Stimmventil der Verbindung mit einem Tracheostoma dient, und einen regenerativen Filter aufweist zum Feuchtigkeits- und Wärmeaustausch beim Atmen durch das Stimmventil, sowie ein Gehäuse, das den Filter aufnimmt, und eine erste Öffnung an einer Seite von dem Filter zur Verbindung mit dem Tracheostoma sowie zumindest eine zweite Öffnung einer gegenüberliegenden Seite von dem Filter, die mit der Umgebung verbunden ist, und ein manuell betätigbares Ventilelement zum Blockieren eines Luftdurchganges durch den Filter, wobei eine Hülse in das Innere von dem Gehäuse ragt und einen Ventilsitz bildet, welcher die erste Öffnung definiert, um mit dem Ventilelement durch eine manuelle Betätigung von diesem abdichtend zum Eingriff zu kommen. Das dort offenbarte Stimmventil ist relativ kompliziert aufgebaut und bedient sich einer Feder als Rückstellmechanismus beziehungsweise eines verformbaren Deckelteiles als Ventilelement.

DE 20 2011 003 781 U1 offenbart eine Beatmungsvorrichtung mit einer Halterung und einem regenerativen Wärme-Feuchtigkeitsaustauscher für Patienten mit Luftröhrenschnitt, wobei aus einem Gehäuse in einem Abstand ein Filter, gesichert durch eine Arretierung, stirnseitig mit einer Feuchtigkeit und Luft undurchlässigen Klebefolie bestückt, bei Ausüben des Schließdrucks durch Verschluss der Öffnung den Sprachvorgang sowie bei Nachlassen durch die Filterfederung den seitlichen Luftweg ermöglicht.

WO 2010/060983 A1 offenbart eine Atemschutzausrüstung mit einem Filterkörper, welcher einen Innenraum, gebildet durch ein Gehäuse und ein Ventilelement, vollständig ausfüllt, wobei der Filterkörper als Rückstellfeder eingesetzt ist und ein Ventilteil durch einen umlaufenden Bund in einer distalen Bewegung desselben gehindert ist.

Aufgabe der vorliegenden Erfindung ist es daher, ein Sprechventil für Laryngektomierte oder Tracheotomierte zur Verfügung zu stellen, welches einfach aufgebaut ist.

Diese Aufgabe wird gelöst durch ein erfindungsgemäßes Sprechventil für Laryngektomierte oder Tracheotomierte mit einem Gehäuse mit einer proximalen, ersten Öffnung und einer distalen, zweiten Öffnung, einen von dem Gehäuse zumindest teilweise umgebenen, einteiligen Filter aus einem elastischen Material mit einem proximalen ersten Ende und einem distalen zweiten Ende, wobei der Filter über eine Stirnfläche des Gehäuses an der distalen zweiten Öffnung teilweise hervorsteht, wobei mindestens ein Verbreiterungsmittel an einer Außenwandung des Gehäuses angeordnet ist, wobei eine distale Öffnung des Verbreiterungsmittels bündig mit der Stirnfläche des Gehäuses abschließt und wobei ein Deckel mit zumindest einem Teilbereich seiner unteren Fläche mit einer Oberseite des Filters an dessen distalem zweiten Ende fest verbunden und wobei der Filter über ein an einer Innenwandung des Gehäuses angeordnetes Haltemittel gehalten ist, wobei ein Durchmesser d₁ des Deckels größer ist als Durchmesser d₂ des Filters, so dass ein eine seitliche Filterwandung des Filters überragender Rand gebildet ist, wobei durch Anwendung von Druck auf den Deckel aus einer Ausgangsstellung eine untere Fläche des Randes zumindest teilweise mit der Stirnfläche des Gehäuses zusammenwirkt zur Erzeugung einer Schließstellung des Sprechventiles, und wobei der Deckel mit dem Filter bei Entlastung in die Ausgangstellung zurückkehrt. Insbesondere ist der eine seitliche Filterwandung des Filters überragende Rand des Deckels an seiner unteren Fläche ringförmig umlaufend ausgebildet. Bevorzugt beträgt der Überstand des Filters über die Stirnfläche des Gehäuses etwa 5 % bis etwa 50 %, bevorzugt etwa 8 % bis etwa 30 %, noch weiter bevorzugt etwa 10 % bis etwa 20 % der Gesamthöhe des Filters, wobei die Gesamthöhe im nichtkomprimierten Zustand des Filters ausgehend von dessen unterer, das proximale erste Ende abschließende Fläche und die das distale zweite Endes definierende obere Fläche des Filters bestimmt ist.

"Proxmales Ende" oder "proximale Öffnung" im Sinne der vorliegenden Erfindung bezeichnet das beziehungsweise die der Körpermitte des Nutzers des erfindungsgemäßen Stimmventiles zugewandte Ende beziehungsweise Öffnung, wohingegen im Sinne der vorliegenden Erfindung mit "distalem Ende" beziehungsweise "distale Öffnung" bezeichnet ist das beziehungsweise die der Körpermitte des Nutzers entferntere Ende beziehungsweise Öffnung des Gehäuses beziehungsweise des Filters.

Das erfindungsgemäße Sprechventil weist den großen Vorteil auf, dass dieses ausgesprochen einfach hergestellt werden kann und damit sehr kostengünstig ist. Gleichzeitig kann der Nutzer auf einfache Art und Weise ein Sprechen bei Verschließen des Sprechventiles durch Ausübung eines Druckes auf den Deckel aus einer Ausgangsstellung in eine Schließstellung erzeugen. Der Filter dient dabei sowohl der Filterung und Anfeuchtung der Atemluft als auch als elastisches Mittel, mittels welchem das Sprechventil aus einer Schließstellung wieder in eine Ausgangsstellung zurückkehrt. Das elastische Material ist dabei vorteilhafterweise aus Kunststoff gebildet, wobei weiter vorteilhafterweise der Kunststoff eine schwammartige Struktur mit einer Vielzahl von Poren und Kanälen aufweist, wobei diese bevorzugt vollkommen ungerichtet sind. Jedoch können in dem Filterkörper selbst zusätzlich oder allein angeordnete und ausgerichtete Kanäle vorgesehen sein.

Die Luft dringt zumindest über eine Außenwandung des Filters im Bereich zwischen der Stirnfläche des Gehäuses und der unteren Fläche des Randes des Deckels in den Filter ein und wird umgelenkt auf die distale Öffnung des Gehäuses beziehungsweise das distale Ende des Filters zu. In umgekehrter Weise geht ausgeatmete Luft durch den Filter in dem erfindungsgemäßen Sprechventil hindurch. Aber auch ein Luftdurchgang durch mindestens eine vorzugsweise mittig am Deckel angeordnete Öffnung ohne jegliche Umlenkung ist möglich.

Der Filter ist mit dem Deckel fest verbunden. Die Überführung in eine Schließstellung erfolgt ausschließlich über den Deckel, wobei zugleich eine Komprimierung des Filters erfolgt. Der Filter selbst wird dabei in dem die Stirnfläche überstehenden Teil ausschließlich durch den Deckel geführt. Mithin liegt der Deckel nicht bloß am Filter an. Denn dann würde er sich lösen und eine Schließung des Sprechventils wäre kaum mehr möglich.

Zwischen Gehäuse und Deckel kann außerhalb des Körpers des Filters mindestens ein, bevorzugt mindestens zwei, drei, vier, fünf, sechs oder mehr elastische Elemente, verbunden mit dem Deckel und/oder dem Gehäuse, insbesondere im Bereich zwischen der distalen Stirnfläche des Gehäuses und dem auf der unteren Fläche des Deckels ausgebildeten Rand, angeordnet sein, beispielsweise in Form von elastischen Kunststoffbändern, die bei Überführung in eine Schließstellung sich nach außen biegen und bei Loslassen aus der Schließstellung den Filter in die Ausgangs(Offen-)-Stellung zurückbewegen, dabei unterstützend den Filter. Bevorzugt weist das erfindungsgemäße Sprechventil jedoch keine Verbindungen zwischen dem Gehäuse und dem Deckel auf. Hierdurch erfolgt eine Komprimierung und Führung des Filters allein über den Deckel, der fest mit dem Filter verbunden ist. Der Filter erfährt damit keine zusätzliche Führung in dem über die Stirnfläche hervorstehenden Teil derselben, diese erfolgt alleinig durch den Deckel. In einer weiter bevorzugten Ausführungsform kann im Bereich der distalen zweiten Öffnung ein Schutzelement angeordnet sein, welches zumindest teilweise den über die Stirnfläche überstehenden Teil des Filters im Bereich von dessen Außenwandung schützt. Dabei kann das Schutzelement bevorzugt an einer Außenwandung des Gehäuses, weiter bevorzugt im distalen Endbereich desselben, angeordnet sein. Bevorzugt weist das Schutzelement mindestens eine Öffnung auf, um den Luftdurchtritt über die Außenwandung des Filters zu ermöglichen. Es ist vorzugsweise ringförmig ausgebildet, mit einem unteren, an der Außenwandung des Gehäuses angeordneten, bevorzugt fest mit dieser verbundenen Ringteil und einem oberen Ringteil, welcher im Bereich der Außenwandung des Filters und mithin oberhalb, aber nicht über der distalen Stirnfläche des Gehäuses, angeordnet ist. Bevorzugt ist der obere Ringteil mit seiner Oberseite zumindest etwas unterhalb dem distalen zweiten Ende des Filters angeordnet. Der untere und der obere Ringteil sind bevorzugt mittels mindestens zwei Stegen miteinander verbunden, zwischen denen Öffnungen angeordnet sind für den Durchtritt von Luft. Der untere und/oder obere Ringteil weist einen Innendurchmesser auf, der nur wenig größer ist als der Durchmesser d₁ des Deckels. Das Schutzelement ist nicht mit dem Deckel verbunden. Es verleiht dem Filter bei Überführung des Sprechventils in eine Schließstellung keine Führung. Sowohl das Schutzelement als auch elastische Element verhindern ein zufälliges Verschließen des Sprechventiles, insbesondere im Schlaf.

Vorteilhafterweise wird als elastisches Material für den Filter ein Kunststoff eingesetzt, ausgewählt aus einer Gruppe umfassend Polyurethane, bevorzugt aus der Gruppe der Polyurethanweichschaumstoffe. Das Kunststoffmaterial ist vorzugsweise als Schaumstoff ausgebildet, bevorzugt mit einer Zellenzahl in einem Bereich von 5,5 bis 7,5 (1/cm). Vorzugsweise beträgt die Rohdichte des Schaumstoffs 20 bis 26 kg/m³, gemessen gemäß DIN EN ISO 845. Bevorzugt liegt die Stauchhärte CV40 gemäß DIN EN ISO 3386-1 in einem Bereich von etwa 2,9 bis etwa 5 kPa für den Schaumstoff. Die Zugfestigkeit gemäß DIN 53571 A für den Schaumstoff liegt vorzugsweise in einem Bereich von etwa 55 bis etwa 200 kPa, vorzugsweise beträgt sie mindestens 58 kPa. Die Bruchdehnung gemäß DIN 53571 A des Schaumstoffs beträgt mindestens 90 %, bevorzugt liegt sie in einem Bereich von etwa 95 % bis etwa 200 %.

Erfindungsgemäß ist der Filter über mindestens ein an einer Innenwandung des Gehäuses angeordnetes Haltemittel gehalten, bevorzugt ist dieses Haltemittel beabstandet von der proximalen, ersten Öffnung des Gehäuses angeordnet. Das Haltemittel ist vorteilhafterweise zumindest teilweise als umlaufender Bund, stegförmig und/oder als sich zur Mitte des Gehäuses hin erstreckender, insbesondere verjüngender Vorsprung, ausgebildet. Unter dem Begriff stegförmig fallen im Sinne der vorliegenden Erfindung auch beispielsweise sternförmige Ausbildungen, beispielsweise drei-, vier-, fünf-, sechs- oder achtarmige Sterne, wobei jeder Arm durch einen Steg gebildet ist. Der Filter ist bevorzugt mit seiner proximalen Oberfläche beabstandet von der proximalen ersten Öffnung des Gehäuses angeordnet. Hierdurch kann ein Filter mit geringerer Bauhöhe zum Einsatz gelangen. Insbesondere durch das Hervorragen über die Stirnfläche des Gehäuses kann der Filter gleichwohl trotz Volumenreduzierung aufgrund der vergrößerten angebotenen Oberfläche seine Wärme- und Feuchtigkeitstauscher-Funktion erfüllen.

Vorteilhafterweise ist der Filter mit dem Haltemittel durch Klebung, Verschweißung oder Verhakung verbunden. Beispielsweise könnte bei einer stegförmigen Ausgestaltung als Kreuz mit vier Armen (Stegen) dieses Kreuz nahe oder an einer Stirnfläche der proximalen ersten Öffnung des Gehäuses des erfindungsgemäßen Sprechventiles angeordnet sein, und der Filter mit seiner unteren, dieser proximalen ersten Öffnung zugewandten proximalen ersten Ende beziehungsweise unteren Fläche mittels Klebung mit der dieser Fläche des Filters zugewandten Oberfläche des Kreuzes verbunden sein. Es könnte jedoch auch vorgesehen sein, dass beispielsweise umlaufend an der Innenwandung, sei es nahe der proximalen Öffnung des Gehäuses, sei es etwas entfernt von dieser, Haltemittel, beispielsweise wiederum in Form eines umlaufenden Randes, stegförmig, einschließlich sternförmig in jeglicher Art, und/oder als sich zur Mitte des Gehäuses hin erstreckender, insbesondere verjüngender, Vorsprung, vorgesehen sind. Diese können dann allein oder zusätzlich zu einem an der proximalen Öffnung des Gehäuses angeordneten weiteren Haltemittel, beispielweise einem Steg, einschließlich einem sternförmig ausgebildeten, vorgesehen sein. Sind die Haltemittel als sich zur Mitte des Gehäuses hin verjüngender Vorsprung ausgebildet, sind diese bevorzugt spitzkegelförmig ausgebildet, so dass diese in das elastische Material des Filters eingreifen können. Es sind vorteilhafterweise mindestens zwei, drei, vier, fünf, sechs oder acht derartiger Haltemittel an der Innenwandung des Gehäuses, eingreifend in das elastische Material des Filters, angeordnet, bevorzugt gleichmäßig über die Innenwandung verteilt, weiter bevorzugt in einer gemeinsamen Ebene.

Der zumindest teilweise umlaufende Rand kann auch Unterbrechungen aufweisen, so dass hierdurch durch dessen obere, dem proximalen Ende des Filters zugewandte Oberfläche in einer Ebene liegend eine Auflage für den Filter zur Verfügung gestellt ist. Es können beispielsweise zwei, drei, vier oder mehr kreisbogenförmige Ringabschnitte an der Innenwandung des Gehäuses in einer Ebene vorgesehen sein. Bei Verbindung dieser kreisbogenförmigen Ringabschnitte durch Verbreiterung derselben würde dann ein umlaufender Rand geschaffen. Auf dem zumindest teilweise umlaufenden Rand liegt der Filter auf. Der umlaufende Rand kann mit seiner dem proximalen Ende des Filters zugewandten Oberfläche beispielsweise mit einem Kleber versehen sein, der eine Verbindung zum elastischen Material des Filters zur Verfügung stellt, oder aber es kann auch eine Verschweißung mit dem Filtermaterial dort erfolgen. Auch eine Verhakung mit dem Filtermaterial kann durch Anordnung von Haken, die in das elastische Material des Filters hineinragen, auf der dem proximalen Ende des Filters zugewandten Oberfläche des zumindest teilweise umlaufenden Randes erfolgen. Der zumindest teilweise umlaufende Rand ist vorzugsweise nahe dem proximalen Ende des Gehäuses angeordnet, und kann ebenso wie die Stege, einschließlich sternförmig angeordneter, mit der unteren, von dem Gehäuse wegweisenden Fläche bündig mit seiner unteren proximalen Fläche mit einer proximalen Stirnfläche des Gehäuses abschließen.

Vorteilhafterweise weist im Sinne der vorliegenden Erfindung das Gehäuse ein am proximalen ersten Ende angeordnetes, luftdurchlassendes Bodenteil auf. Das Bodenteil kann dabei auch gleichzeitig Haltemittel in oben wiedergegebenen Sinne sein, insbesondere wenn dieses zumindest teilweise als umlaufender Rand oder aber stegförmig, einschließlich sternförmig, ausgebildet ist.

Vorteilhafterweise ist der Deckel mit dem Filter verklebt, verschweißt oder verhakt verbunden, besonders bevorzugt verklebt. Die Verklebung muss dabei nicht über die gesamte distale Oberfläche des Filters erfolgen, sondern kann auch nur über einen Teilbereich, ebenso eine Verschweißung oder Verhakung, erfolgen.

Vorteilhafterweise ist vorgesehen, dass der auf der unteren Fläche des Deckels außerhalb der äußeren Umfangsfläche des Filters angeordnete Rand des Deckels ringförmig ausgebildet ist und dieser zumindest die Stirnfläche der distalen Öffnung des Gehäuses abdeckt, weiter bevorzugt etwas über diese übersteht, mithin über eine Außenkontur des Gehäuses, gebildet durch die Umfangsfläche desselben, hervorsteht. Dieser Überstand beträgt maximal das Doppelte der Breite der ringförmigen Stirnfläche der distalen Öffnung des Gehäuses, und liegt bevorzugt in einem Bereich von etwa dem 1,1-fachen bis etwa 1,9-fachen der Breite der Stirnfläche der distalen Öffnung des Gehäuses.

Erfindungsgemäß ist mindestens ein Verbreiterungsmittel an einer Außenwandung des Gehäuses angeordnet. Besonders bevorzugt ist das Verbreiterungsmittel als Bund, vorzugsweise umlaufender Bund, ausgebildet. Das Verbreiterungsmittel kann jedoch auch als unterbrochener Bund ausgestaltet sein, so dass dann mehrere Verbreiterungsmittel an der Außenwandung des Gehäuses für den Deckel angeordnet sind. Das Verbreiterungsmittel ist erfindungsgemäß derart an der Außenwandung des Gehäuses angeordnet, dass eine distale, das heißt körperferne Oberfläche desselben bündig mit der Stirnfläche des Gehäuses abschließt. Durch das Verbreiterungsmittel ist vorteilhafterweise die Auflage beziehungsweise Kontaktfläche des Gehäuses für den Deckel vergrößert, wodurch in Schließstellung eine Unterbrechung des Luftstromes verbessert wird mittels und/oder durch Inkontaktbringung mit der unteren Fläche des ringförmig ausgebildeten Randes des Deckels.

In einer bevorzugten Ausführungsform sind eine Außenkontur der unteren Fläche des Randes des Deckels und eine Außenkontur der Stirnfläche des Gehäuses und/oder des Verbreiterungsmittels, angeordnet auf der Außenwandung des Gehäuses, aneinander angepasst ausgebildet. Dies kann beispielsweise dadurch erfolgen, dass die untere Fläche des Randes und die Stirnfläche des Gehäuses und/oder des Verbreiterungsmittels Oberflächen aufweisen, die parallel und eben zueinander ausgebildet sind. Jedoch ist auch eine Ausgestaltung mit einer anderen Außenkontur der unteren Fläche des Randes und der Stirnfläche des Gehäuses und/oder des Verbreiterungsmittels möglich. Durch eine Anpassung der Ausbildung der Außenkontur der unteren Fläche des Randes und der Stirnfläche des Gehäuses kann vorteilhafterweise die Bedienung des erfindungsgemäßen Sprechventiles verbessert werden. So kann durch die genannte Anpassung eine Zentrierung des Deckels in Schließstellung erfolgen, so dass eine grundsätzlich mögliche Bewegung des Deckels mitsamt dem mit diesem verbundenen Filter in Richtung einer Ebene parallel zu der Stirnfläche des Gehäuses unterbunden ist. Durch eine derartige wie vorgenannte Bewegung wird eine Verschiebung des ringförmigen Randes des Deckels derart, dass dieser nicht mehr mit der Stirnfläche des Gehäuses zumindest in einem Teilbereich zusammenwirken kann, vermieden.

In einer bevorzugten Ausführungsform ist auf der Stirnfläche und/oder dem Verbreiterungsmittel an der Außenwandung des Gehäuses eine erste Erhöhung angeordnet. Die Erhöhung kann auch teilweise auf der Stirnfläche und teilweise auf dem Verbreiterungsmittel, angeordnet an der Außenwandung des Gehäuses, angeordnet sein. Die erste Erhöhung kann jede beliebige Ausgestaltung aufweisen. Beispielsweise kann in einer Ausführungsform das Verbreiterungsmittel, angeordnet an der Außenwandung des Gehäuses, als zumindest teilweise umlaufender Bund ausgebildet sein, wobei jedoch dessen distale, das heißt dem Deckel zugewandte Oberfläche nicht bündig mit der Stirnfläche des Gehäuses abschließt, sondern über diese in Richtung auf den Deckel vorsteht. Die Oberfläche der Erhöhung kann, im Schnitt durch eine Ebene senkrecht durch diese betrachtet, verschiedenste Konturen aufweisen. Beispielsweise kann diese viereckig ausgebildet sein, oder auf einer Seite, vorzugsweise der dem Gehäuseinnern zugewandten Seite eine Abschrägung aufweisen, jedoch auch aus Kreisbogensegmenten zumindest teilweise ausgebildet sein auf der dem Gehäuseinnern zugewandten Seite, und dabei beispielsweise auch eine sigmoidal ausgebildete Oberfläche aufweisen.

In einer weiter bevorzugten Ausführungsform ist auf einer Unterseite des Randes des Deckels eine zweite Erhöhung angeordnet. Diese zweite Erhöhung ist vorzugsweise auf der unteren Fläche des ringförmigen Randes des Deckels, die mit der Stirnfläche und/oder dem mindestens einen Verbreiterungsmittel, angeordnet in einer Außenwandung des Gehäuses, zusammenwirkt, angeordnet. Diese zweite Erhöhung kann dabei in ihrer Außenkontur der ersten Erhöhung, angeordnet auf der Stirnfläche und/oder dem Verbreitungsmittel des Gehäuses, angepasst sein, und weist beispielsweise im Querschnitt gesehen in einer Ebene senkrecht durch die zweite Erhöhung und in einer Ebene mit einer Mittelachse durch das Gehäuse liegend, eine viereckige Ausgestaltung auf, wobei vorzugsweise auf einer der ersten Erhöhung des Gehäuses zugeordneten Oberfläche der Erhöhung diese auch eine Abschrägung aufweisen kann. Auch kann die Oberfläche der zweiten Erhöhung aus Kreisbogensegmenten zumindest teilweise gebildet sein, und beispielsweise eine sigmoidal ausgebildete Oberfläche aufweisen.

Im Sinne der vorliegenden Erfindung kann eine Anpassung der Außenkontur der unteren Fläche des Randes des Deckels und der Außenkontur der Stirnfläche des Gehäuses aneinander auch derart erfolgen, dass die Stirnfläche beispielsweise schräg zum Gehäuseinnern abfallend ausgebildet ist, also ohne eine erste Erhöhung, so dass eine entsprechend ausgebildete (zweite) Erhöhung, angeordnet auf der unteren Fläche des ringförmigen Randes des Deckels, und in ihrer Außenkontur angepasst der Abschrägung der Stirnfläche des Gehäuses in das Gehäuseinnere, mit dieser zusammenwirkt. Bei einer derartigen Ausgestaltung weist die Stirnfläche keine Erhöhung, sondern eine Ausnehmung oder eine Aussparung auf, die mit einer (zweiten) Erhöhung auf der unteren Fläche des ringförmigen Randes des Deckels zusammenwirkt.

In einer besonders bevorzugte Ausführungsform der vorliegenden Erfindung ist auf der unteren Fläche des Randes des Deckels und/oder der Stirnfläche beziehungsweise auf dem mindestens einen Verbreiterungsmittel des Gehäuses mindestens ein Zentriermittel angeordnet. Das mindestens eine Zentriermittel ist dabei vorteilhafterweise auf der unteren Fläche des ringförmigen Randes des Deckels, der außerhalb des Außenumfanges beziehungsweise -wandung des Filters liegt, angeordnet. Das Zentriermittel dient ebenfalls wie die Anpassung der Außenkontur der unteren Fläche des Randes des Deckels und der Außenkontur der Stirnfläche des Gehäuses aneinander einer verbesserten Handhabung des erfindungsgemäßen Sprechventiles. Ein erstes Zentriermittel ist beispielsweise zapfenförmig ausgestaltet angeordnet auf der unteren Fläche des ringförmigen Randes. Dabei ist bevorzugt vorgesehen, dass mehrere solcher zapfenförmig ausgebildeter Zentriermittel angeordnet sind auf der unteren Fläche des ringförmigen Randes des Deckels, beispielsweise mindestens zwei, drei, vier, fünf, sechs, acht oder mehr. Das zapfenförmige Zentriermittel kann dabei im Querschnitt gesehen eine beliebige geometrische Ausgestaltung aufweisen, und ist im einfachsten Fall im Querschnitt gesehen viereckig ausgebildet, es kann jedoch auch im Querschnitt gesehen dreieckig ausgebildet sein, und beispielsweise auch als Spitzkegel ausgestaltet sein. Dieses mindestens eine erste Zentriermittel kann beispielsweise in mindestens ein zweites Zentriermittel, angeordnet auf der Stirnfläche des Gehäuses und/oder im Bereich eines Verbreiterungsmittels, angeordnet an der Außenwandung des Gehäuses, eingreifen. In diesem Fall kann beispielsweise die Gehäusewandung ausgehend von der distalen Stirnfläche vorteilhafterweise der Außenkontur des ersten Zentriermittels angepasste Ausnehmungen als zweites Zentriermittel aufweisen. Jedoch können entsprechende Aussparungen auch beispielsweise im Verbreiterungsmittel, angeordnet auf der Außenwandung des Gehäuses, angeordnet sein. Dabei kann die Ausnehmung oder Aussparung, die das zweite Zentriermittel bildet, das erste Zentriermittel vollständig, oder aber auch nur teilweise aufnehmen. Im letzteren Fall ist das zweite Zentriermittel als Aussparung anzusprechen, die beispielsweise im Bereich einer Innenwandung des Verbreiterungsmittel, zugewandt dem Gehäuseinnern, oder aber im Bereich der Innenwandung des Gehäuses, zugewandt dem Inneren des Gehäuses, und ausgehend von der distalen Stirnfläche desselben dort angeordnet, angeordnet ist. Bei einer vollständigen Aufnahme des ersten Zentriermittels im zweiten Zentriermittel ist das zweite Zentriermittel als Bohrung oder Ausnehmung anzusprechen.

In einer weiter bevorzugten Ausführungsform ist auf der unteren Fläche des Randes, insbesondere der unteren Fläche des ringförmig ausgebildeten Randes des Deckels, und/oder der Stirnfläche beziehungsweise auf dem mindestens einen Verbreiterungsmittel zumindest teilweise eine Dichtung angeordnet. Die Dichtung kann dabei auch auf der ersten und/oder zweiten Erhöhung, die auf der Stirnfläche und/oder dem Verbreiterungsmittel, angeordnet an der Außenwandung des Gehäuses, beziehungsweise auf der unteren Fläche des Randes Deckels beziehungsweise der dort angeordneten zweiten Erhöhung angeordnet sein.

Die Dichtung kann aus jedem geeigneten Material hergestellt sein, das medizinisch unbedenklich ist, beispielsweise aus einem geeigneten Silikonmaterial oder Ähnlichem.

Vorteilhafterweise ist die Dichtung einstückig mit dem Gehäuse beziehungsweise Deckel ausgebildet, das heißt, diese ist fest, gegebenenfalls aber lösbar, mit dem Gehäuse beziehungsweise dem Deckel verbunden. Die Verbindung kann dabei vorteilhafterweise durch Klebung, aber auch durch Koextrusion oder durch sonstige Verbindungsarten erfolgen.

Diese und weitere Vorteile der vorliegenden Erfindung werden anhand der nachfolgenden Figuren näher erläutert. Es zeigen:
- Fig. 1:: einen Querschnitt entsprechend Schnitt A - A gemäß Fig. 3 durch eine erste Ausführungsform des erfindungsgemäßen Sprechventiles in Ausgangsstellung;
- Fig. 2:: eine Einzelheit der ersten Ausführungsform des erfindungsgemäßen Sprechventiles gemäß Fig. 1 in Schließstellung zum Erzeugen einer Sprechfunktion im Querschnitt;
- Fig. 3:: eine Unteransicht der ersten Ausführungsform des erfindungsgemäßen Sprechventiles gemäß Fig. 1;
- Fig. 4:: eine vergrößerte Querschnittdarstellung der Ausbildung der Gehäusewandung im Bereich der distalen Stirnfläche in einer zweiten Ausführungsform des erfindungsgemäßen Sprechventiles;
- Fig. 5:: eine vergrößerte Querschnittdarstellung der Ausbildung der Gehäusewandung im Bereich der distalen Stirnfläche in einer dritten Ausführungsform des erfindungsgemäßen Sprechventiles;
- Fig. 6:: eine vergrößerte Querschnittdarstellung der Ausbildung der Gehäusewandung im Bereich der distalen Stirnfläche in einer vierten Ausführungsform des erfindungsgemäßen Sprechventiles;
- Fig. 7:: eine Querschnittsdarstellung betreffend die Ausgestaltung des Deckelteiles mit einer an die Ausgestaltung der distalen Stirnfläche angepassten Außenkontur zu der vierten Ausführungsform gemäß Fig. 6;
- Fig. 8:: eine Querschnittsdarstellung einer Ausgestaltung des Gehäuses mit distaler Stirnfläche mit einem zweiten Zentriermittel in einer fünften Ausführungsform des erfindungsgemäßen Sprechventiles;
- Fig. 9:: eine Querschnittsdarstellung des Deckelteiles mit am Rand angeordneten ersten Zentriermittel in der fünften Ausführungsform des erfindungsgemäßen Sprechventiles in Übereinstimmung mit Fig. 8; sowie
- Fig. 10:: eine Draufsicht auf die distale Stirnfläche des Gehäuses mit zweiten Zentriermitteln gemäß der fünften Ausführungsform des erfindungsgemäßen Sprechventiles in Übereinstimmung mit Fig. 8.

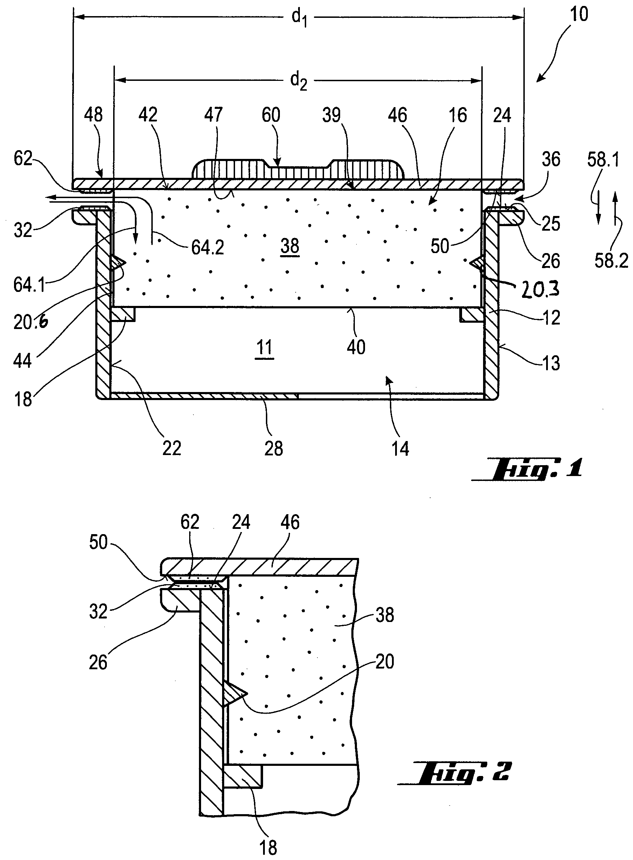

Fig. 1 zeigt ein insgesamt mit den Bezugszeichen 10 bezeichnetes erfindungsgemäßes Sprechventil für Laryngektomierte oder Tracheotomierte mit einem Gehäuse 12, welches in einen hier nicht gezeigten Halter, beispielsweise einer Basisplatte mit einem Pflaster, die am Hals des Patienten getragen wird, oder einer Trachealkanüle, verbunden werden kann. Das Gehäuse 12 weist eine Außenwandung 13 sowie eine proximale erste Öffnung 14, die körpernah dem Patienten angeordnet ist, und eine distale zweite Öffnung 16, die körperfern bezogen auf den Patienten angeordnet ist, auf. Im Gehäuse 12 ist zum überwiegenden Teil angeordnet ein Filter 38 mit einer Seitenwandung 44 aus einem offenporigen Kunststoffmaterial, welches elastisch ausgebildet ist. Das Gehäuse 12 weist eine distale Stirnfläche 24 auf. An der Außenwandung 13 ist im Bereich der distalen zweiten Öffnung 16 des Gehäuses 12 ein Verbreiterungsmittel 26 in Form eines umlaufenden Bundes angeordnet, dessen Oberfläche 25 bündig mit der Stirnfläche 24 des Gehäuses 12 angeordnet ist.

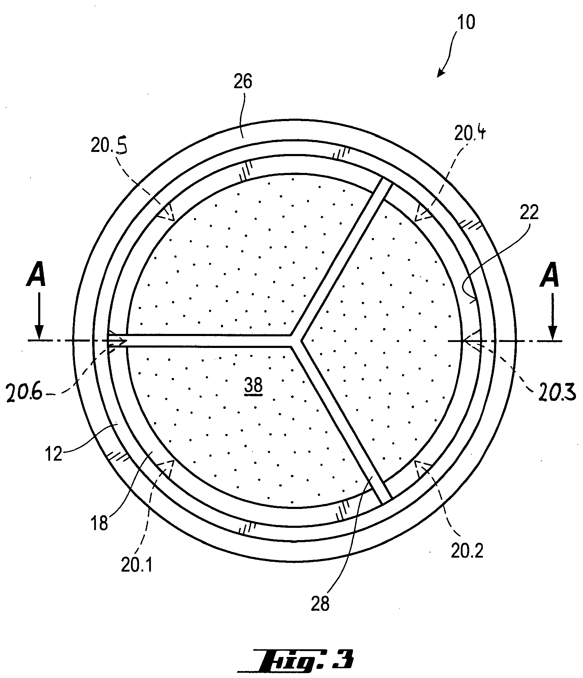

An einer Innenwandung 22 des Gehäuses 12 angeordnet ist ein Haltemittel 18, ausgebildet als umlaufender Bund, wobei die distale Oberseite desselben in Kontakt kommen kann beziehungsweise kommt mit einer proximalen Oberfläche 40 des Filters 38. Weiterhin sind an der Innenwandung 22 des Gehäuses 12 insgesamt sechs weitere Haltemittel 20.1 bis 20.6 (siehe Fig.3) angeordnet, welche spitzkegelförmig ausgebildet sind und mit ihrem spitzen Ende in das elastische Material des Filters 38 eingreifen. Dabei sind diese weiteren Haltemittel 20 gleichmäßig verteilt über die Innenwandung 22 des Gehäuses 12 und zwar in einer Ebene, senkrecht zu einer gedachten Mittelachse des Filters gelegen und in etwa parallel beispielsweise zur proximalen Oberfläche 40 des Filters 38 ausgerichtet, angeordnet. Vorteilhafterweise sind mindestens zwei weitere, bevorzugt drei, noch weiter bevorzugt vier, fünf, sechs oder acht oder mehr derartige weitere Haltemittel 20 an der Innenwandung 22 des Gehäuses 12 angeordnet. Die weiteren Haltemittel 20 verhindern ein Herausrutschen des Filters 38 aus einem Inneraum 11 des Gehäuses 12. Alternativ und im Sinne der vorliegenden Erfindung könnte jedoch auch vorgesehen sein, dass die weiteren Haltemittel 20 weggelassen werden, und stattdessen das Haltemittel 18 auf seiner distalen Oberseite beispielsweise mit hakenförmigen Mitteln oder aber zumindest teilflächig, bevorzugt ganzflächig mit einem Klebemittel versehen ist, welche beziehungsweise welches eine feste Verbindung mit dem elastischen Material des Filters 38 zur Verfügung stellt/stellen. Hierdurch wird eine Bewegung des Filters 38 aus dem Gehäuseinnern 11 hinaus vermieden.

Des Weiteren weist das Gehäuse 12 ein Bodenteil 28 auf, welches, wie Fig. 3 entnommen werden kann, sternförmig mit drei Armen ausgebildet ist. Die Stege des Bodenteiles 28 setzen an der Innenwandung 22 des Gehäuses 12 an. Vorteilhafterweise ist eine proximale Oberfläche des Bodenteiles 28 in etwa bündig mit einer proximalen Stirnfläche des Gehäuses 12 ausgebildet. In einer alternativen Ausführungsform der vorliegenden Erfindung wäre es beispielsweise möglich, dass das Bodenteil 28 sogleich als Haltemittel 18 beziehungsweise 20 wirkt. Dann könnte beispielsweise eine Ausführungsform dergestalt vorliegen, dass der Filter 38 verbunden ist mit der distalen Oberfläche des Bodenteiles 28, zumindest teilweise, beispielsweise durch Anbringung eines Klebemittels auf dieser oder Vorsehung von Verhakungsmitteln, die in das elastische Material des Filters 38 eingreifen. Dann könnten die Haltemittel 18 und 20 weggelassen werden, Haltemittel 20 könnten jedoch gleichwohl vorgesehen sein, wobei diese dann derart ausgebildet sein sollten, dass diese in das elastische Material des Filters 38 eingreifen.

Des Weiteren weist das Sprechventil 10 gemäß Fig. 1 einen Deckel 46 auf, der eine untere Fläche 47 aufweist, an welcher der Filter 38 mit seinem distalen Ende 42 mit seiner Oberfläche 39 zumindest teilweise, bevorzugt vollständig, verbunden ist, beispielsweise durch Verschweißung, Verklebung oder durch Vorsehung entsprechender Verhakungsmittel. Dabei kann bei Vorsehung eines Klebemittels dieses auch nur in einem Teilbereich zwischen dem distalen Ende 42 mit der Oberfläche 39 des Filters 38 und der unteren Fläche 47 des Deckels 46 angeordnet sein, solange hierdurch ein hinreichend fester Halt des Deckels 46 am Filterkörper 38 zur Verfügung gestellt ist. Der Deckel 46 besteht im Wesentlichen aus einer ebenen Platte, die beispielsweise aus einem Kunststoffmaterial gebildet sein kann, welches vorteilhafterweise relativ hart ausgebildet ist, jedoch gleichwohl eine gewisse Biegsamkeit aufweist, um letztendlich in Schließstellung des Sprechventiles 10 eine sichere Unterbrechung des Luftstromes zur Verfügung zu stellen. Auf der distalen Oberseite des Deckels 46 ist in etwa mittig ein Bedienmittel 60 angeordnet, welches für den Patienten das Ertasten des richtigen Druckpunktes zur Erzielung einer optimalen Schließwirkung zur Unterbrechung des Luftstromes zur Erzeugung eines Sprechens erleichtert.

Der Deckel 46 weist einen Rand 48 mit einer unteren Fläche 50 auf. Die untere Fläche 50 kann als ringförmige Fläche angesprochen werden, der Rand 48 als ringförmig ausgebildet.

Der Filter 38 steht zu etwa 15 % seiner Gesamthöhe über der Stirnfläche 24 des Gehäuses 12 hervor. Wird nun von einem Patienten Druck auf den Deckel 46 in Richtung auf die proximale erste Öffnung14 des Gehäuses 12 ausgeübt, so wird der Filter 38 komprimiert, und die untere Fläche 50 des Randes 48 des Deckels 46 kommt in Kontakt mit der Stirnfläche 24 als auch der distalen Oberfläche 25 des Verbreiterungsmittels 26. Hierdurch wird ein Luftstrom von außerhalb des Gehäuses, der beispielsweise durch einen Pfeil 64.1 symbolisiert wird, in das Gehäuseinnere 11 ebenso wie ein Luftstrom in umgekehrter Richtung, symbolisiert durch einen Pfeil 64.2, unterbunden.

Im Sinne der vorliegenden Erfindung kann es beispielsweise auch möglich sein, dass im Bereich des Bedienmittels 60 zentral eine Öffnung vorliegt, so dass auch Luft durch diese Öffnung vom Patienten eingeatmet werden kann und in das Innere 11 des Gehäuses 12 eindringt beziehungsweise in umgekehrter Richtung das Sprechventil 10 wieder verlassen kann. Durch die Unterbrechung des Luftstromes durch das Inkontaktbringen des Deckels mit dem Gehäuse ist dem Patienten ein Sprechen ermöglicht. Fig. 1 zeigt das Sprechventil 10 in der Ausgangsstellung, das heißt einer Stellung, in welcher dem Patienten ein Atmen ermöglicht ist, indem Luft durch eine seitliche Öffnung 36 und gegebenenfalls eine im Deckel vorgesehene zentrale Öffnung im Bereich des Bedienmittels 60 in das Gehäuseinnere 11 des Gehäuses 12 einströmen und wieder ausströmen kann. Die Öffnung 36 ist dabei kreisförmig umlaufend ohne Unterbrechungen ausgebildet.

Zur Verbesserung des Inkontaktbringens und damit auch der Unterbrechung des Luftstromes sind am Deckelteil 46 und am Gehäuse 12 Dichtmittel 32 beziehungsweise 62 angeordnet. Das Dichtmittel 32 ist dabei auf der Stirnfläche 24 des Gehäuses 12 als auch der distalen Oberfläche 25 des Verbreiterungsmittels 26 angeordnet. Entsprechend gegenüberliegend auf der unteren Fläche 50 des Randes 48 des Deckels 46 ist das Dichtmittel 62 angesprochen. Sowohl das Dichtmittel 32 als auch das Dichtmittel 62 sind aus Silikon oder aber einem hinreichend weichen, das heißt elastischen Kunststoff hergestellt, welches beziehungsweise welcher medizinverträglich ausgestattet ist.

Fig. 2 zeigt die Schließstellung des Sprechventiles 10 gemäß Fig. 1 im Detail, und dabei insbesondere das Inkontaktbringen der Dichtmittel 32 und 62.

Fig. 3 zeigt eine Unteransicht der ersten Ausführungsform des Sprechventiles 10 gemäß Fig. 1, aus welcher insbesondere die Ausgestaltung des Bodenteils 28 als dreiarmiger Stern und dessen Angreifen an der Innenwandung 22 des Gehäuses 12 erkennbar ist. Die radialsymmetrische Ausgestaltung des Sprechventiles 10 ist ebenfalls Fig. 3 gut zu entnehmen, ebenso wie die sechs Haltemittel 20.1, 20.2, 20.3, 20.4, 20.5 und 20.6..

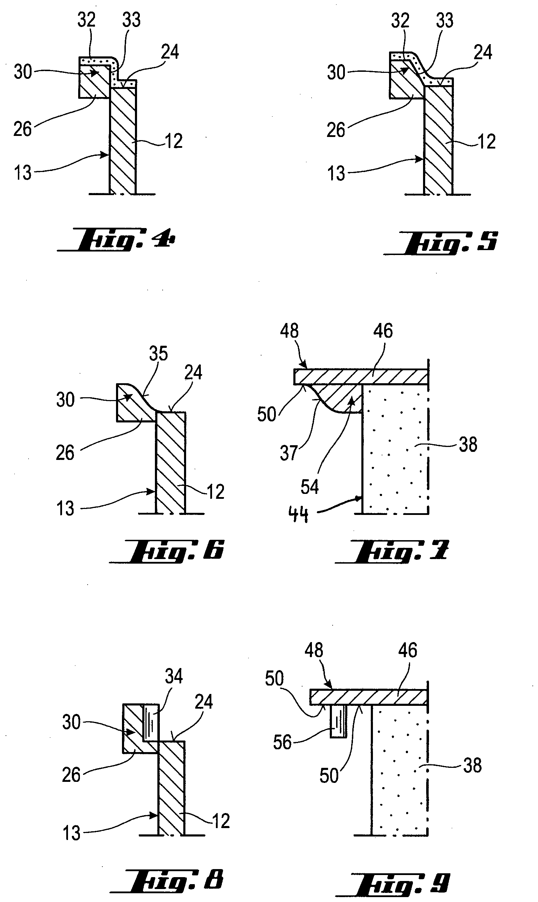

Fig. 4 bis Fig. 6 zeigen nun eine zweite, dritte und vierte Ausführungsform des Sprechventiles 10 in einer Detailansicht betreffend den distalen Endbereich des Gehäuses 12. Dabei sind gleiche Teile mit gleichen Bezugszeichen wie bei der ersten Ausführungsform gemäß Fig. 1 bis Fig. 3 bezeichnet. Fig. 4 zeigt ein Gehäuse 12 mit einem an einer Außenwandung 13 desselben angeordneten Verbreiterungsmittel 26, welches eine erste Erhöhung 30 aufweist. Diese ist im Querschnitt gesehen etwa viereckig, nahezu quadratisch, ausgebildet. Auf der hierdurch gebildeten distalen Oberfläche der ersten Erhöhung 30 und auf der Stirnfläche 24 des Gehäuses 12 ist ein Dichtmittel 32 angeordnet, wobei dieses auch an einem zwischen der ersten Erhöhung 30 und der distalen Stirnfläche 24 des Gehäuses 12 im Bereich eines dort angeordneten Versprungs 33 angeordnet ist.

Fig. 5 zeigt eine dritte Ausführungsform des erfindungsgemäßen Sprechventiles 10 mit einem Gehäuse 12 und einem an einer Außenwandung 13 desselben angeordneten Verbreiterungsmittel 26, welches eine erste Erhöhung 30 aufweist, wobei diese im Unterschied zu der zweiten Ausführungsform gemäß Fig. 4 einen abgeschrägten Versprung 33 aufweist, zugewandt zu einem hier nicht näher gezeigten Gehäuseinnern 11 des Gehäuses 12. Der schräg ausgebildete Versprung 33 verläuft dabei ausgehend von der distalen Oberseite der ersten Erhöhung 30 abfallend auf die distale Stirnfläche 24 des Gehäuses 12 zu. Ein Dichtmittel 32 ist über die gesamte, dem hier nicht gezeigten Deckel 46 zugewandte Oberfläche der ersten Erhöhung 30 und der distalen Stirnfläche 24 des Gehäuses 12 angeordnet.

Fig. 6 zeigt eine vierte Ausführungsform des erfindungsgemäßen Sprechventiles 10 mit einem Gehäuse 12 und einem an einer Außenwandung 13 desselben angeordneten Verbreiterungsmittel 26 mit einer ersten Erhöhung 30. Die erste Erhöhung 30 weist dabei im Querschnitt gesehen eine Oberfläche mit einem in etwa sigmoidalen Verlauf einer gekrümmten Komponente 35, welche einem hier nicht gezeigten Deckel 46 zugewandt ist, auf, die in eine distale Stirnfläche 24 des Gehäuses 12 übergeht.

Fig. 7 zeigt zugehörig zur vierten Ausführungsform der vorliegenden Erfindung eine Ausgestaltung eines Deckels 46 mit einer zweiten Erhöhung 54, die an einer unteren Fläche 50 eines Randes 48 des Deckels 46 angeordnet ist, und im Querschnitt gesehen eine gekrümmte Komponente 37 aufweist mit einem in etwa sigmoidalen Verlauf zumindest über einen Teilbereich dieser zweiten Erhöhung 54, wobei nahe einem Filter 38 beziehungsweise dessen Filterseitenwandung 44 ein mehr oder weniger ebener Verlauf dieser Fläche vorliegt, der in etwa der distalen Stirnfläche 24 des Gehäuses 12 entspricht. Fig. 6 und Fig. 7 verdeutlichen, dass eine Anpassung der Außenkontur der unteren Fläche 50 des Deckels 46 und eine Außenkontur der Stirnfläche 24 des Gehäuses 12 als auch eine Außenkontur der ersten Erhöhung 36 und der zweiten Erhöhung 54 vorliegt. Hierdurch erfolgt bei Ausübung eines Druckes aus der Ausgangsstellung auf den Deckel 46 durch den Patienten eine Zentrierung des Deckels 46 mit dem Filter 38 im Gehäuse 12 und damit eine sichere Unterbrechung der Luftdurchfuhr zur Erzielung eines Sprechens.

Fig. 8 zeigt in einer fünften Ausführungsform im Detail eine Ausgestaltung des distalen Endbereiches des Gehäuses 12 mit einer distalen Stirnfläche 24 und einem an einer Außenwandung 13 des Gehäuses 12 angeordneten Verbreiterungsmittel 26, welches eine erste Erhöhung 30 trägt. Diese erste Erhöhung weist Aussparungen 34 auf, die in etwa halbkreisförmig ausgebildet sind.

Fig. 9 zeigt eine hierzu komplementäre Ausbildung eines Deckels 46 für diese fünfte Ausführungsform mit einer unteren Fläche 50 eines Randes 48 mit an dieser angeordneten stiftförmigen Zentriermitteln 56, welche durch die Aussparungen 34 im distalen Endbereich des Gehäuses 12 aufgenommen werden.

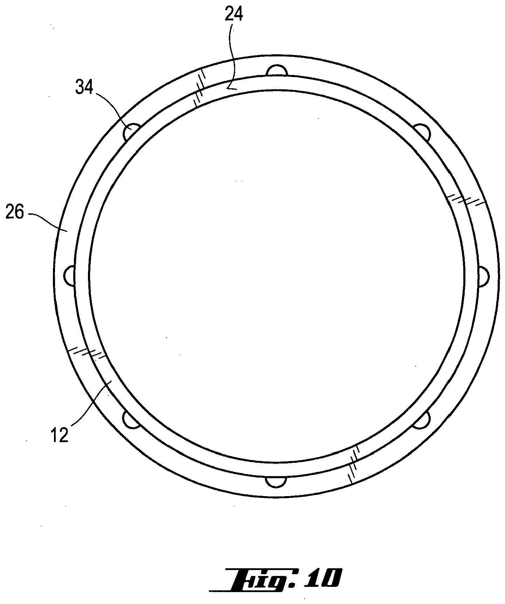

Fig. 10 verdeutlicht in einer Draufsicht auf das Gehäuse 12 gemäß Fig. 8, dass insgesamt acht Aussparungen 34 halbkreisförmig im Bereich des Verbreiterungsmittels 26 mit der ersten Erhöhung 30 vorgesehen sind, in welche eine entsprechende Anzahl von stiftförmigen Zentriermitteln 56, angeordnet am Deckelteil 46, eingreifbar sind. Die Aussparung 34 kann dabei als erstes Zentriermittel im Sinne der vorliegenden Erfindung angesprochen werden, das stiftförmige Zentriermittel 56 als zweites Zentriermittel, wobei das erste und das zweite Zentriermittel zusammenwirken zur Erzielung einer Zentrierung. Die Vorsehung erster und zweiter Zentriermittel 34 beziehungsweise 56 ist auch mit den Ausgestaltungen gemäß Fig. 4 bis Fig. 6 der zweiten bis vierten Ausführungsform kombinierbar, und kann selbstverständlich auch mit der ersten Ausführungsform kombiniert werden.

Abschließend sei darauf hingewiesen, dass die in den Figuren dargestellten Ausgestaltungen der ersten bis fünfte Ausführungsform des erfindungsgemäßen Sprechventiles nicht beschränkend auszulegen sind. Vielmehr können die dort beschriebenen Merkmale untereinander und mit den vor der Figurenbeschreibung beschriebenen Merkmalen der vorliegenden Erfindung zur weiteren Ausgestaltung kombiniert werden. Des Weiteren sei darauf hingewiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen sollen. Es sei insbesondere darauf hingewiesen, dass die in Fig. 1 gezeigte erste Ausführungsform, wie beschrieben, auch anders ausgebildet sein kann, und das insbesondere das Bodenteil 28 auch als Haltemittel 18 beziehungsweise 20 dienen kann, so dass letztendlich auch eine Verkürzung der Bauhöhe des erfindungsgemäßen Sprechventiles erzielbar ist.

## Patentansprüche

1. Sprechventil (10) für Laryngektomierte oder Tracheotomierte mit einem Gehäuse (12) mit einer proximalen, ersten Öffnung (14) und einer distalen, zweiten Öffnung (16), einem von dem Gehäuse (12) zumindest teilweise umgebenen Filter (38) aus einem elastischen Material mit einem proximalen ersten Ende (40) und einem distalen zweiten Ende (42), wobei der Filter (38) über eine Stirnfläche (24) des Gehäuses (12) an der distalen zweiten Öffnung (16) teilweise hervorsteht, wobei mindestens ein Verbreiterungsmittel (26) an einer Außenwandung (13) des Gehäuses (12) angeordnet ist, wobei eine distale Oberfläche (25) des Verbreiterungsmittels (26) bündig mit der Stirnfläche (24) des Gehäuses (12) abschließt, und wobei ein Deckel (46) mit zumindest einem Teilbereich seiner unteren Fläche (47) mit einer Oberseite (39) des Filters (38) an dessen distalem zweiten Ende (42) fest verbunden und wobei der Filter (38) über mindestens ein an der Innenwandung (22) des Gehäuses (12) angeordnetes Haltemittel (18, 20) gehalten ist, wobei ein Durchmesser d₁ des Deckels (46) größer ist als ein Durchmesser d₂ des Filters (38), so dass ein eine seitliche Filterwandung (44) des Filters (38) überragender Rand (48) gebildet ist, wobei durch Anwendung von Druck auf den Deckel (46) aus einer Ausgangsstellung eine untere Fläche (50) des Randes (48) zumindest teilweise mit der Stirnfläche (24) des Gehäuses (12) zusammenwirkt zur Erzeugung einer Schließstellung des Sprechventiles (10), und wobei der Deckel (46) mit dem Filter (38) bei Entlastung in die Ausgangsstellung zurückkehrt.

2. Sprechventil gemäß Anspruch 1, **dadurch gekennzeichnet, dass** der Filter (38) über das mindestens eine an der Innenwandung (22) des Gehäuses (12) angeordnete Haltemittel (18, 20), beabstandet von der proximalen, ersten Öffnung (14) des Gehäuses (12), gehalten ist.

3. Sprechventil gemäß Anspruch 2, **dadurch gekennzeichnet, dass** das Haltemittel (18, 20) zumindest teilweise als umlaufender Bund (18), stegförmig und/oder als zur Mitte des Gehäuses (12) hin ausgerichteter Vorsprung (20) ausgebildet ist.

4. Sprechventil gemäß einem der vorhergehenden Ansprüche, **dadurch gekennzeichnet, dass** das Gehäuse (12) ein an der proximalen ersten Öffnung (14) angeordnetes, luftdurchlassendes Bodenteil (28) aufweist.

5. Sprechventil gemäß einem der Ansprüche 2 bis 4, **dadurch gekennzeichnet, dass** der Deckel (46) mit dem Filter (38) verklebt, verschweißt oder verhakt verbunden ist.

6. Sprechventil gemäß einem oder mehreren der vorhergehenden Ansprüche, **dadurch gekennzeichnet, dass** das Verbreiterungsmittel (26) ein umlaufender Bund ist.

7. Sprechventil gemäß einem der vorhergehenden Ansprüche, **dadurch gekennzeichnet, dass** eine Außenkontur der unteren Fläche (50) des Randes (48) des Deckels (46) und eine Außenkontur der Stirnfläche (24) des Gehäuses (12) und/oder des Verbreiterungsmittels (26) aneinander angepasst ausgebildet sind.

8. Sprechventil gemäß einem der vorhergehenden Ansprüche, **dadurch gekennzeichnet, dass** auf der Stirnfläche (24) und/oder dem Verbreiterungsmittel (26) eine erste Erhöhung (30) angeordnet ist.

9. Sprechventil gemäß einem der vorhergehende Ansprüche, **dadurch gekennzeichnet, dass** auf der unteren Fläche (50) des Randes (48) des Deckels (46) eine zweite Erhöhung (54) angeordnet ist.

10. Sprechventil gemäß einem der vorhergehenden Ansprüche, **dadurch gekennzeichnet, dass** auf der unteren Fläche (50) des Randes (48) des Deckels (46) und/oder der Stirnfläche (24) beziehungsweise auf dem mindestens einen Verbreiterungsmittel (26) des Gehäuses (12) mindestens ein Zentriermittel (34, 56) angeordnet ist.

11. Sprechventil gemäß einem der vorhergehenden Ansprüche, **dadurch gekennzeichnet, dass** auf der untere Fläche (50) des Randes (48) des Deckels (46) und/oder der Stirnfläche (24) beziehungsweise auf dem mindestens einen Verbreiterungsmittel (26) zumindest teilweise eine Dichtung (32, 62) angeordnet ist.

12. Sprechventil gemäß Anspruch 11, **dadurch gekennzeichnet, dass** die Dichtung (32, 62) einstückig mit dem Gehäuse (12) beziehungsweise Deckel (46) ausgebildet ist.

## Claims

1. Speaking valve (10) for laryngectomized and tracheotomized persons with a housing (12) with a proximal, first opening (14) and a distal, second opening (16), a filter (38) which is at least partially surrounded by the housing (12), which filter is made of an elastic material, with a proximal first end (40) and a distal second end (42), wherein the filter (38) partially protrudes beyond an end face (24) of the housing (12) at the distal second opening (16), wherein at least one enlarging aid (26) is arranged at the outer wall (13) of the housing (12), wherein the distal surface (25) of the enlarging aid (26) closes flush with the end face (24) of the housing (12), and wherein a cover (46) is firmly connected by means of at least one partial region of its bottom surface (47) to a upper side (39) of the filter (38) at its distal second end (42), and wherein the filter (38) is held by at least one fixing aid (18, 20) arranged at the inner wall (22) of the housing (12), wherein a diameter d1 of the cover (46) is greater than a diameter d2 of the filter (38), so that an edge (48) is formed that is protruding beyond a lateral filter wall (44) of the filter, wherein by application of pressure onto the cover (46) from an initial position, a lower surface (50) of the edge (48) at least partially interacts with the end face (24) of the housing (12) in order to produce a closed position of the speaking valve (10), and wherein the cover (46) together with the filter (38) returns to the initial position when unloaded.

2. Speaking valve according to claim 1, **characterized in that** the filter (38) is held by the at least one fixing aid (18, 20) on the inside wall (22) of the housing, this fixing aid being arranged at some distance to the proximal first opening (14) of the housing (12).

3. Speaking valve according to claim 2, **characterized in that** the fixing aid (18, 20) is at least partially arranged as a circulating flange (18), in the shape of a ridge and/or as a protrusion (20) aligned towards the center of the housing (12).

4. Speaking valve according to one of the preceding claims, **characterized in that** the housing (12) comprises an air permeable bottom part (28) that is arranged at the proximal first opening (14).

5. Speaking valve according to one of claims 2 to 4, **characterized in that** the cover (46) is attached to the filter (38) by gluing, welding or catching.

6. Speaking valve according to one of the preceding claims, **characterized in that** the enlarging aid (26) is a circular flange.

7. Speaking valve according to one of the preceding claims, **characterized in that** an outer contour of the lower surface (50) of the edge (48) of the cover (46) and an outer contour of the end face (24) of the housing (12) and/or of the enlarging aid (26) are adapted in such a way, that they fit into each other.

8. Speaking valve according to one of the preceding claims, **characterized in that** a first elevation (30) is arranged on the end face (24) and/or on the enlarging aid (26).

9. Speaking valve according to one of the preceding claims, **characterized in that** a second elevation (54) is arranged on a lower surface (50) of the edge (48) of the cover (46).

10. Speaking valve according to one of the preceding claims, **characterized in that** at least one centering aid (34, 56) is arranged on the lower surface (50) of the edge (48) of the cover (46) and/or of the end face (24) or on the at least one enlarging aid (26) of the housing (12).

11. Speaking valve according to one of the preceding claims, **characterized in that** at least a partial sealing gasket (32, 62) is arranged on the lower surface (50) of the edge (48) of the cover (46), and/or the end face (24) or on the at least one enlarging aid (26).

12. Speaking valve according to claim 11, **characterized in that** the sealing gasket (32), 62) is produced in one piece together with the housing (12) or cover (46).

## Revendications

1. Valve de phonation (10) pour laryngectomisés ou trachéotomisés avec un boîtier (12) possédant une première ouverture proximale (14) et une deuxième ouverture distale (16), et un filtre (38) en matériau élastique au moins partiellement entouré par le boîtier (12) et possédant une première extrémité proximale (40) et une deuxième extrémité distale (42), dans laquelle le filtre (38) dépasse partiellement d'une face frontale (24) du boîtier (12) au niveau de la deuxième ouverture distale (16), dans laquelle au moins un moyen d'élargissement (26) est disposé sur une paroi extérieure (13) du boîtier (12), dans laquelle une surface distale (25) du moyen d'élargissement (26) affleure la face frontale (24) du boîtier (12), dans laquelle un couvercle (46) est relié de manière fixe par au moins une partie de sa face inférieure (47) avec une face supérieure (39) du filtre (38) à sa deuxième extrémité distale (42) et le filtre (38) est maintenu par au moins un moyen de maintien (18, 20) disposé sur la paroi intérieure (22) du boîtier (12), sur laquelle un diamètre d1 du couvercle (46) est supérieur au diamètre d2 du filtre (38) de sorte qu'une bordure (48) dépassant une paroi de filtre latérale (44) du filtre (38) est formée, dans laquelle, par l'application d'une pression sur le couvercle (46) à partir d'une position de départ, une face inférieure (50) de la bordure (48) interagit au moins partiellement avec la face frontale (24) du boîtier (12) pour atteindre une position de fermeture de la valve de phonation (10), et dans laquelle le couvercle (46) revient dans la position de départ avec le filtre (38) lors du relâchement.

2. Valve de phonation selon la revendication 1, **caractérisée en ce que** le filtre (38) est maintenu écarté de la première ouverture proximale (14) du boîtier (12) par l'au moins un moyen de maintien (18, 20) disposé sur la paroi intérieure (22) du boîtier (12).

3. Valve de phonation selon la revendication 2, **caractérisée en ce que** le moyen de maintien (18, 20) est configuré au moins partiellement comme un collet périphérique (18), comme une barrette et/ou comme une saillie (20) orientée vers le milieu du boîtier (12).

4. Valve de phonation selon une des revendications précédentes, **caractérisée en ce que** le boîtier (12) présente une partie de fond (28) perméable à l'air disposée sur la première ouverture proximale (14).

5. Valve de phonation selon une des revendications 2 à 4, **caractérisée en ce que** le couvercle (46) est collé, soudé ou accroché au filtre (38).

6. Valve de phonation selon une ou plusieurs des revendications précédentes, **caractérisée en ce que** le moyen d'élargissement (26) est un collet périphérique.

7. Valve de phonation selon une des revendications précédentes, **caractérisée en ce qu'**un contour extérieur de la face inférieure (50) de la bordure (48) du couvercle (46) et un contour extérieur de la face frontale (24) du boîtier (12) et/ou du moyen d'élargissement (26) sont adaptés l'un à l'autre.

8. Valve de phonation selon une des revendications précédentes, **caractérisée en ce qu'**une première élévation (30) est disposée sur la face frontale (24) et/ou le moyen d'élargissement (26).

9. Valve de phonation selon une des revendications précédentes, **caractérisée en ce qu'**une deuxième élévation (54) est disposée sur la face inférieure (50) de la bordure (48) du couvercle (46).

10. Valve de phonation selon une des revendications précédentes, **caractérisée en ce qu'**au moins un moyen de centrage (34, 56) est disposé sur la face inférieure (50) de la bordure (48) du couvercle (46) et/ou sur la face frontale (24) ou sur l'au moins un moyen d'élargissement (26) du boîtier (12).

11. Valve de phonation selon une des revendications précédentes, **caractérisée en ce qu'**un joint (32, 62) est disposé au moins partiellement sur la face inférieure (50) de la bordure (48) du couvercle (46) et/ou sur la face frontale (24) ou sur l'au moins un moyen d'élargissement (26).

12. Valve de phonation selon la revendication 11, **caractérisée en ce que** le joint (32, 62) est formé d'une pièce avec le boîtier (12) ou le couvercle (46).
